# EUROPEAN PATENT APPLICATION

(11) **EP 4 756 835 A1**
(43) Date of publication of application: **10.06.2026**
(21) Application number: 23948573.3
(22) Date of filing: 14.08.2023
(51) Int. Cl.: G16H 50/20, G16H 50/50, G16H 10/60, G16H 50/70

(54) **PREDICTIVE ANALYSIS METHOD FOR DISEASE PROGRESS MONITORING AND MANAGEMENT AND DEVICE FOR PERFORMING SAME**

(30) Priority: 04.08.2023 KR 20230102205
(71) Applicant: WELT Corp., Ltd, Seocho-gu Seoul 06628 (KR)
(72) Inventor: KANG, Seong Ji, Seoul 06366 (KR); ROH, Hye Kang, Seoul 05507 (KR); KIM, Joo Young, Seoul 04014 (KR); LEE, Do Hyun, Yongin-si Gyeonggi-do 16826 (KR); JEONG, Hwa Young, Incheon 21451 (KR)
(74) Representative: AWA Sweden AB
(86) International application number: PCT/KR2023/012014
(87) International publication number: WO 2025/033578

(57) **Abstract**

The present invention relates to a disease prediction and disease analysis method for disease management, and a device for performing the method. The disease prediction and disease analysis method for disease management may comprise the steps in which a disease management device: receives user data; and generates user disease management data on the basis of the user data.

## Description

### BACKGROUND

### 1. Field of the Invention

The present invention relates to a predictive analysis method for disease progression monitoring and management and an apparatus for performing the method, and more particularly, to a predictive analysis method for disease progression monitoring and management, which monitors the progression of a user's disease and manages the user's disease and an apparatus for performing the method.

### 2. Discussion of Related Art

With the development of various smart technologies, data of personal daily activities is recorded, and individual life can be efficiently managed on the basis of the recorded data. In the meantime, health-related data logging is attracting attention due to the increasing interest in healthcare. Many users have already been generating and utilizing various health-related data including data on exercise, diet, sleep, and the like through user devices such as smartphones, wearable devices, and the like. In the past, health-related data was generated and managed only by medical institutions, but now users have begun to generate and manage their own health-related data through user devices such as smartphones and wearable devices.

In many cases, health-related data logging is performed through a wearable device. A wearable device is a user device that is carried by or attached to a user. Due to the development of Internet of things (IoT) and the like, wearable devices are frequently used for collecting health-related data. A wearable device may collect a user's physical change information and surrounding data of the user through equipment and provide advice required for the user's healthcare on the basis of the collected data.

A user's health-related data may include a user biomarker, and research is ongoing on a method of making a medical prescription adaptively to a user on the basis of the user's health-related data.

The technology was developed through the 'R&D for Digital Healthcare Demonstration and Adoption in Medical Institutions in 2023 (RS-2023-00266002) "Multi-Center Clinical Validation and RWE Generation for Insurance Coverage of a Digital Therapeutic for Insomnia" by the Korea Health Industry Development Institute.

As related art, there is Korean Patent No. 10-2425479.

### SUMMARY OF THE INVENTION

The present invention is directed to providing a predictive analysis method for disease progression monitoring and management and an apparatus for performing the method, which may manage a user's disease by performing disease prediction and disease monitoring through an artificial intelligence (Al)-based disease prediction model and disease monitoring model.

The present invention is also directed to providing a predictive analysis method for disease progression monitoring and management and an apparatus for performing the method, which may train various AI models for disease prediction and disease monitoring and replace the AI model for disease prediction and disease monitoring through performance comparison of the various AI models, thereby achieving the most accurate disease prediction and monitoring.

According to an aspect of the present invention, there is provided a disease prediction and analysis method for disease management, the method comprises receiving, by a disease management device, user data and generating, by the disease management device, user disease management data based on the user data.

Meanwhile, the disease management data includes disease prediction data for a user or disease monitoring data for the user, the disease prediction data is generated based on a disease prediction artificial intelligence (AI) model, and the disease monitoring data is generated based on a disease monitoring AI model.

Further, the user data is preprocessed into user data (disease prediction) and input into the disease prediction AI model, the user data is preprocessed into user data (disease monitoring) and input into the disease monitoring AI model, and the user data (disease prediction) and the user data (disease monitoring) are time-series data.

According to another aspect of the present invention, there is provided a disease management device for performing disease prediction and disease analysis for disease management, the disease management device being implemented to receive user data, and generate user disease management data based on the user data.

Meanwhile, the disease management data includes disease prediction data for a user or disease monitoring data for the user, the disease prediction data is generated based on a disease prediction AI model, and the disease monitoring data is generated based on a disease monitoring AI model.

Further, the user data is preprocessed into user data (disease prediction) and input into the disease prediction AI model, the user data is preprocessed into user data (disease monitoring) and input into the disease monitoring AI model, and the user data (disease prediction) and the user data (disease monitoring) are time-series data.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a conceptual diagram illustrating a disease management system for disease prediction and disease analysis according to an embodiment of the present invention.
FIG. 2 is a conceptual diagram illustrating the operation of a disease management device according to an embodiment of the present invention.
FIG. 3 is a conceptual diagram illustrating the operation of a disease management device according to an embodiment of the present invention.
FIG. 4 is a conceptual diagram illustrating the operation of a disease management device according to an embodiment of the present invention.
FIG. 5 is a conceptual diagram illustrating a method of preprocessing user data for disease management and disease monitoring according to an embodiment of the present invention.
FIG. 6 is a conceptual diagram illustrating a method of determining a disease prediction time-series unit according to an embodiment of the present invention.
FIG. 7 is a conceptual diagram illustrating a method of determining a disease monitoring time-series unit according to an embodiment of the present invention.
FIG. 8 a conceptual diagram illustrating an AI model generation platform for generating an AI model according to an embodiment of the present invention.

### DETAILED DESCRIPTION OF EXEMPLARY EMBODIMENTS

The detailed description of the present invention will be made with reference to the accompanying drawings showing examples of specific embodiments of the present invention. These embodiments will be described in detail such that the present invention can be performed by those skilled in the art. It should be understood that various embodiments of the present invention are different but are not necessarily mutually exclusive. For example, a specific shape, structure, and characteristic of an embodiment described herein may be implemented in another embodiment without departing from the scope and spirit of the present invention. In addition, it should be understood that a position or arrangement of each component in each disclosed embodiment may be changed without departing from the scope and spirit of the present invention. Accordingly, there is no intent to limit the present invention to the detailed description to be described below. The scope of the present invention is defined by the appended claims and encompasses all equivalents that fall within the scope of the appended claims. Like reference numerals refer to the same or like elements throughout the description of the figures.

Hereinafter, in order to enable those skilled in the art to practice the present invention, exemplary embodiments of the present invention will be described in detail with reference to the accompanying drawings.

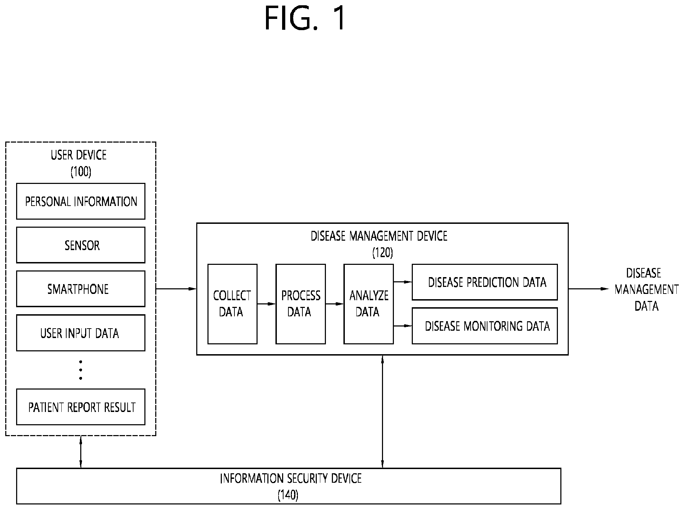

FIG. 1 is a conceptual diagram illustrating a disease management system for disease prediction and disease analysis according to an embodiment of the present invention.

FIG. 1 discloses a disease management system for predicting and analyzing a user's disease based on user data.

Referring to FIG. 1, the disease management system may include a user device 100, a disease management device 120, and an information security device 140.

The user device 100 may be implemented to generate user data for a prescription for the user, transmit the generated user data to the disease management device 120, and receive disease management data adaptively determined according to the user.

The user data may include a variety of data used for managing the user's disease. For example, the user data may include biomarker data obtained through the user device 100 (e.g., a smartphone or a wearable device), input data separately input by the user, and personal information (sex, age, etc.) of the user.

The disease management data may include disease prediction data for a user determined by the disease management device 120, disease monitoring data for a user, and the like. The disease management device 120 may transmit the disease management data through the user device 100.

The disease management device 120 may be implemented to determine user disease management data based on user data. The disease management device 120 may generate disease prediction data for the user through data collection, data processing, and data analysis of collected user data, disease monitoring data, etc., and the disease management data may be generated based on disease prediction data for the user and disease monitoring data for the user.

The disease management device 120 may generate the disease prediction data, the disease monitoring data, and the like based on a disease prediction AI model and a disease monitoring AI model.

The information security device 140 may be implemented to secure information generated from the user device 100 and the disease management device 120.

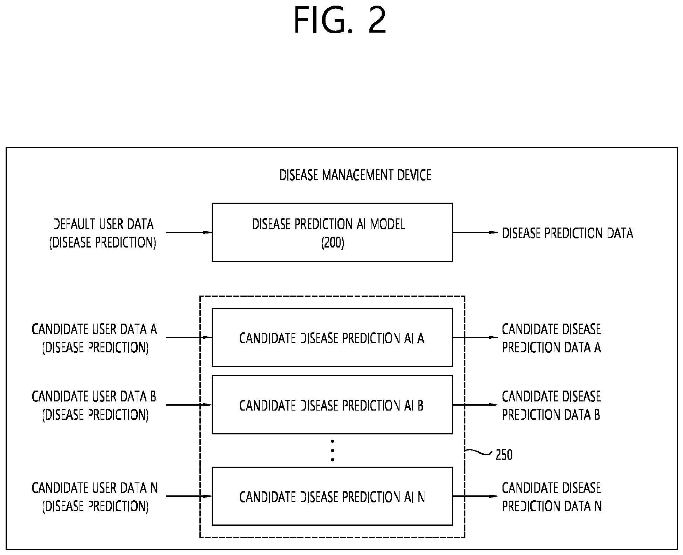

FIG. 2 is a conceptual diagram illustrating the operation of a disease management device according to an embodiment of the present invention.

FIG. 2 discloses a method of generating user disease management data by processing user data in a disease management device. In particular, a disease prediction AI model for generating disease prediction data is specifically disclosed.

Referring to FIG. 2, the disease management device may receive user data, predict a disease based on the user data, and generate disease prediction data. In addition, the disease management device may generate disease monitoring data for monitoring a predicted disease or a confirmed disease.

The disease management device may predict a user's disease based on a disease prediction AI model 200. The disease prediction AI model 200 may be generated based on learning about user data of a user with a disease.

The disease prediction AI model 200 may be trained for predicting multiple diseases or individual diseases. For example, there may be one disease prediction AI model for predicting insomnia, eating disorders, dementia, and the like or individual disease prediction AI models 200 for predicting each of insomnia, eating disorders, and dementia.

The disease prediction AI model 200 may be generated based on learning about user data of the user with a disease.

For example, the disease prediction AI model 200 may learn user data required for diagnosis of a specific disease based on user data of each of a plurality of users having the specific disease. The disease prediction AI model 200 may extract a user data pattern of the specific disease to predict the specific disease.

Hereinafter, a disease prediction method assuming the disease prediction AI model 200 that predicts a specific disease A is disclosed.

First, the disease prediction AI model 200 may be trained using extracted default user data related to disease A primarily. The default user data may be user data determined based on existing medical knowledge about disease A. The disease prediction AI model 200 may be primarily generated based on the default user data.

Next, in the present invention, a plurality of pieces of candidate user data may be generated based on various combinations of the user data, and a plurality of candidate disease prediction AI models 250 may each be generated based on one of the plurality of pieces of candidate user data.

When a specific candidate disease prediction AI model 250 among the plurality of candidate disease prediction AI models 250 has higher performance and higher reliability than the disease prediction AI model 200, the disease prediction Al model 200 may be replaced with the specific candidate disease prediction AI model 250, the specific candidate disease prediction AI model 250 may operate as the disease prediction AI model 200, and candidate user data corresponding to the candidate disease prediction AI model 250 may be set to user data.

For convenience of description, it has been assumed that the disease prediction AI model 200 performs separate prediction for disease A, but the disease prediction AI model 200 can predict a plurality of diseases, and this embodiment may also be included in the scope of the present invention.

The disease prediction AI model 200 determined in the above manner may generate user disease prediction data based on the user data.

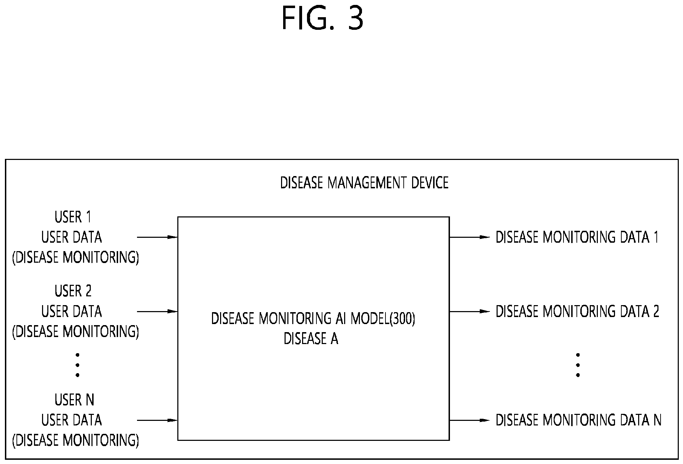

FIG. 3 is a conceptual diagram illustrating the operation of a disease management device according to an embodiment of the present invention.

FIG. 3 discloses a method of generating user disease management data by processing user data in a disease management device. In particular, there is disclosed a disease monitoring AI model for monitoring, in association with a disease prediction AI model, a user disease predicted by the associated disease prediction AI model and a previously confirmed user disease.

Referring to FIG. 3, a disease monitoring AI model 300 may generate disease monitoring data for monitoring a user's disease based on user data (disease monitoring).

The user data (disease monitoring) obtained by pre-processing user data using the disease monitoring AI model 300 may be input. The user data (disease monitoring) may be determined differently for each disease in consideration of the user's disease prediction data and/or the user's disease data.

The disease monitoring AI model 300 may generate disease monitoring data by monitoring the progression of the user's disease by analyzing the user data (disease monitoring) input over time in a time-series manner.

Extraction of the user data for time-series analysis of the user data (disease monitoring) may be determined in consideration of the amount of the user data (disease monitoring) and data characteristics according to the disease. For example, the extent of disease progression for each disease may vary over time. Some diseases might exhibit rapid recovery, while other diseases might exhibit gradual progression over an extended period. Therefore, in consideration of the disease progression, the faster the progression speed of the disease, the shorter the period for analyzing and monitoring the user data (disease monitoring) can become.

In addition, in relation to the amount and characteristics of the user data (disease monitoring), more accurate analysis becomes possible with a relatively larger amount of the user data (disease monitoring) and a relatively higher frequency for inputting the user data (e.g., actual examination results from medical institutions or precise measurement results based on wearable devices) that is also relatively more reliable, whereby the period for analyzing and monitoring the user data (disease monitoring) can be relatively shortened.

The disease monitoring AI model 300 may extract the user data (disease monitoring) for each of a plurality of users having the same disease and learn changes in the diseases of the plurality of users. For example, when users 1 to N are diagnosed with disease A, based on each of user data 1 (disease monitoring) to user data N (disease monitoring), it is possible to learn user data (disease monitoring) about changes until the user's disease disappears, in association with the disease prediction AI model and/or diagnostic results from medical institutions offline.

That is, the disease monitoring AI model 300 may learn user data (disease monitoring) on the process of users recovering from a specific disease, monitor the user's recovery process from the disease, and provide the monitoring results as user disease monitoring data.

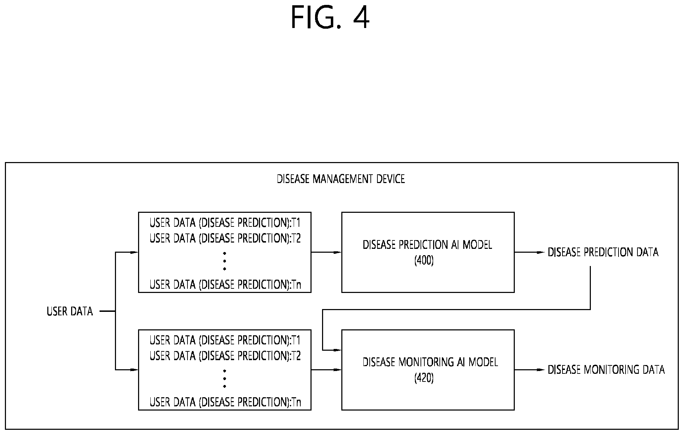

FIG. 4 is a conceptual diagram illustrating the operation of a disease management device according to an embodiment of the present invention.

FIG. 4 discloses a method of generating disease prediction data, disease monitoring data, and the like for a user by processing user data based on a disease prediction AI model and a disease monitoring AI model. For convenience of description, it has been assumed that the disease prediction AI model of the disease management device predicts a plurality of diseases, but each of a plurality of disease prediction AI models can also predict a separate disease, and this embodiment may also be included in the scope of the present invention.

Referring to FIG. 4, when user data is input, the user data may be primarily preprocessed and divided into user data (disease prediction) for disease prediction. For example, user data may be preprocessed into user data 1 (disease prediction) to user data N (disease prediction) for prediction of disease 1 to disease N.

In an embodiment of the present invention, screening may be performed primarily based on the user data, only prediction of diseases likely to occur in the user may be performed, and only user data (disease prediction) for diseases with the probability of occurring in the user greater than or equal to a threshold probability may be preprocessed and generated.

The user data (disease prediction) may be collected in different time-series (or periods) depending on diseases to perform disease prediction for the user. For example, user data (disease prediction) for disease A and user data (disease prediction) for disease B may be accumulated as data of different periods and different time-series, and the user's disease A and disease B may be predicted through a disease prediction AI model 400.

In addition, the user data may be preprocessed and generated as user data (disease monitoring) to be input to a disease monitoring AI model 420.

The user data (disease monitoring) may be adaptively changed according to the monitoring results of the disease. For example, initially, default data for disease monitoring may be generated as the user data (disease monitoring).

Thereafter, data not requiring additional monitoring and data requiring additional monitoring may be determined according to user disease monitoring data, and the user data (disease monitoring) may be adaptively changed according to such determination.

In the present invention, when a corresponding user is predicted to have a specific disease with a probability of occurring in the user equal to greater than a threshold probability using the disease prediction AI model 400, the user data (disease monitoring) may be generated, and disease monitoring may be performed based on the disease monitoring AI model 420.

In addition, for diseases that are already confirmed by online/offline medical institutions, user data (disease monitoring) may be directly generated without the need for the separate disease prediction AI model 400, and disease monitoring may be performed based on the disease monitoring AI model 420.

When monitoring is performed using the disease monitoring AI model 420 based on the disease predicted based on the disease prediction AI model 400, an additional prediction procedure may be performed on the predicted disease. When the probability of the specific disease occurring in the user, which is predicted and monitored based on the disease prediction AI model 400, falls below the threshold probability, the specific disease may be excluded from the monitoring targets.

In the above-described embodiment of the present invention, it has been assumed that both disease prediction and disease monitoring are performed in the disease management device. However, only disease prediction can be performed based on the disease prediction AI model 400 or only disease monitoring can be performed based on the disease monitoring AI model 420, and these embodiments may also be included in the scope of the present invention.

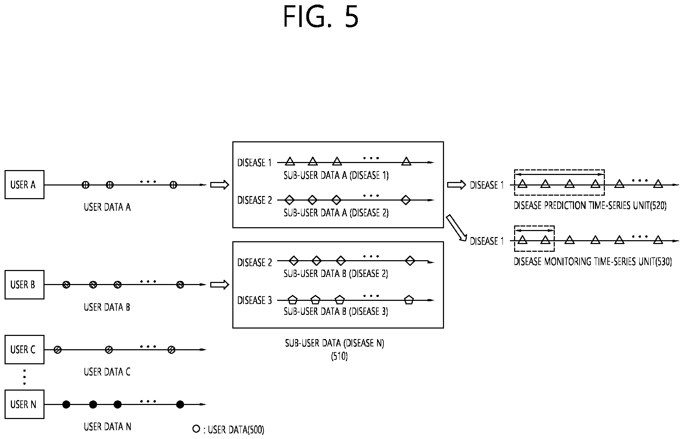

FIG. 5 is a conceptual diagram illustrating a method of preprocessing user data for disease management and disease monitoring according to an embodiment of the present invention.

FIG. 5 discloses a method of preprocessing user data for disease management and disease monitoring.

Referring to FIG. 5, user data 500 is divided for each disease, and the user data 500 divided for each disease may be used for disease monitoring and disease management by constructing time-series unit data as time-series data.

When user A, user B, and user C exist, user data A, user data B, and user data C of each of user A, user B, and user C may be collected as time-series data. The user data 500 may be collected at different periods in consideration of the possibility of obtaining data for each user, and the types of the user data 500 that can be obtained may also be different.

The user data 500 may be divided for each disease in consideration of relevance to the disease and divided into sub-user data (disease N). For example, when user A has disease 1 and disease 2, user data A may be divided into sub-user data A (disease 1) and sub-user data A (disease 2). When user B has disease 2 and disease 3, user data B may be divided into sub-user data B (disease 2) and sub-user data B (disease 3).

The sub-user data (disease N) 510 may be divided into a disease prediction time-series unit 520 for disease prediction and a disease monitoring time-series unit 530 for disease monitoring.

The disease prediction time-series unit 520 and the disease monitoring time-series unit 530 may be adaptively determined in consideration of characteristics of user data (an amount of user data, a period in which user data can be collected, reliability of user data, etc.), disease characteristics (the speed of disease treatment, the presence/absence of a chronic disease, the need to work with offline medical treatment, etc.), disease prediction results, disease monitoring feedback results, and the like.

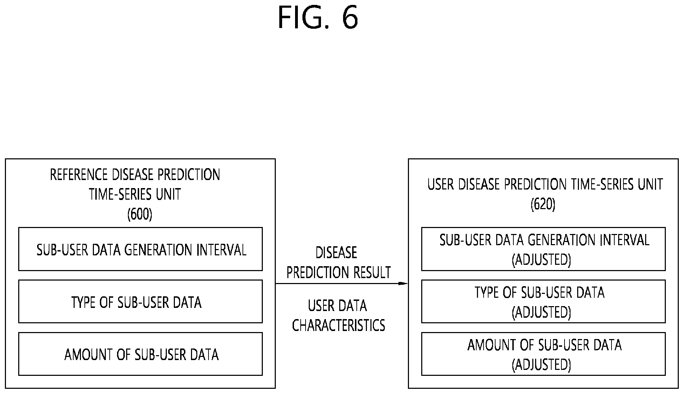

FIG. 6 is a conceptual diagram illustrating a method of determining a disease prediction time-series unit according to an embodiment of the present invention.

FIG. 6 discloses a method of determining a disease prediction time-series unit.

Referring to FIG. 6, in order to determine a disease prediction time-series unit, a minimum data unit for generating a disease prediction result may be determined as a reference disease prediction time-series unit 600. The reference disease prediction time-series unit 600 may be defined in consideration of disease characteristics. In addition, the reference disease prediction time-series unit 600 may be defined to have a disease prediction probability greater than or equal to a threshold probability.

The characteristics of the reference disease prediction time-series unit 600 may be defined based on a generation interval of sub-user data, a type of sub-user data, and an amount of sub-user data. For example, a reference disease prediction time-series unit 600 for predicting a migraine may be defined based on a migraine-related sub-user data generation interval, a type of migraine-related sub-user data, and an amount of migraine-related sub-user data which are defined for migraine prediction.

The reference disease prediction time-series unit 600 may be adjusted in consideration of disease prediction results. In an embodiment of the present invention, the characteristics of the reference disease prediction time-series unit 600 may be changed in consideration of the disease prediction result of the disease prediction AI model. For example, even in a case where the amount of sub-user data is relatively reduced due to an increase in the performance of the disease prediction AI model or the sub-user data generation interval is reduced, when prediction exceeding a threshold probability is possible, the amount of sub-user data that should be included in the reference disease prediction time-series unit 600 may be converted. That is, the characteristics of the reference disease prediction time-series unit 600 may be newly set based on the feedback on the disease prediction result of the disease prediction AI model.

The reference disease prediction time-series unit 600 may be adjusted in consideration of characteristics of the user data, and determined as a user disease prediction time-series unit 620. Along with a relative increase in the reliability of the user data, the amount of the sub-user data of the reference disease prediction time-series unit 600 may be set to be relatively small and the sub-user data generation interval may be set to be relatively short to be defined as the user disease prediction time-series unit 620. In addition, along with a relative reduction in the period for inputting the user data, the sub-user data generation interval may be set to be relatively short to be defined as the user disease prediction time-series unit.

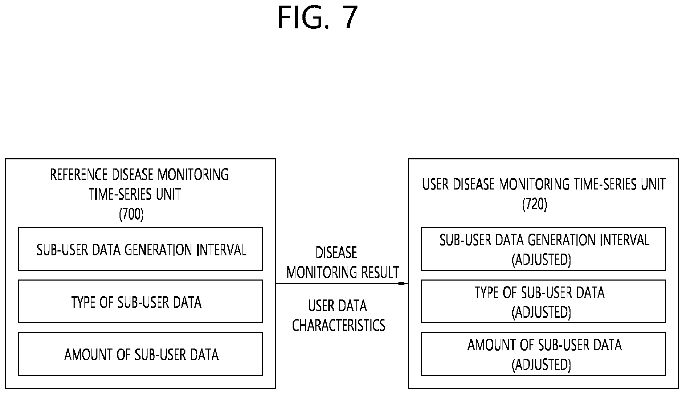

FIG. 7 is a conceptual diagram illustrating a method of determining a disease monitoring time-series unit according to an embodiment of the present invention.

FIG. 7 discloses a method of determining a disease monitoring time-series unit.

Referring to FIG. 7, in order to determine the disease monitoring time-series unit, a minimum data unit for generating a disease monitoring result may be determined as a reference disease monitoring time-series unit 700. The reference disease monitoring time-series unit 700 may be defined in consideration of disease characteristics. In addition, the reference disease monitoring time-series unit 700 may be defined to have disease monitoring performance with accuracy greater than or equal to a threshold probability.

Characteristics of the reference disease monitoring time-series unit 700 may be defined based on a sub-user data generation interval, a type of sub-user data, and an amount of sub-user data. For example, the reference disease monitoring time-series unit 700 for predicting a migraine may be defined based on a migraine-related sub-user data generation interval, a type of migraine-related sub-user data, and an amount of migraine-related sub-user data which are defined for migraine prediction.

The reference disease monitoring time-series unit 700 may be adjusted in consideration of a disease monitoring result. In an embodiment of the present invention, the characteristics of the reference disease monitoring time-series unit 700 may be changed in consideration of the disease monitoring result of the disease monitoring AI model. For example, even in a case where the amount of sub-user data is relatively reduced due to an increase in the performance of the disease monitoring AI model or the sub-user data generation interval is reduced, when prediction exceeding a threshold probability is possible, the amount of sub-user data that should be included in the reference disease monitoring time-series unit 700 may be converted. That is, the characteristics of the reference disease prediction time-series unit 600 may be newly set based on the feedback on the disease monitoring result of the disease monitoring AI model.

The reference disease monitoring time-series unit 700 may be adjusted in consideration of characteristics of the user data, and determined as a user disease monitoring time-series unit 720. Along with a relative increase in the reliability of the user data, the amount of the sub-user data of the reference disease monitoring time-series unit 700 may be set to be relatively small and the sub-user data generation interval may be set to be relatively short to be defined as the user disease monitoring time-series unit 720. In addition, along with a relative reduction in the period for inputting the user data, the sub-user data generation interval may be set to be relatively short to be defined as the user disease monitoring time-series unit 720.

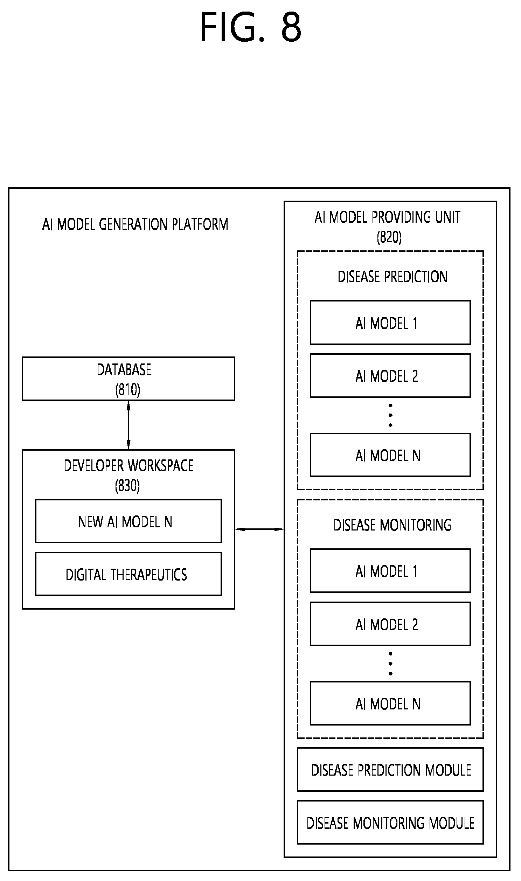

FIG. 8 a conceptual diagram illustrating an AI model generation platform for generating an AI model according to an embodiment of the present invention.

FIG. 8 discloses a method of generating an AI model such as a disease prediction AI model and a disease monitoring AI model by a user (e.g., a developer).

Referring to FIG. 8, the AI model generation platform may include a database 810, an AI model providing unit 820, and a developer workspace 830.

The database 810 may include training data for training an AI model. For example, the database 810 may include user data of users for training AI models such as a disease prediction AI model and a disease monitoring AI model.

The AI model providing unit 820 may be implemented to provide the disease prediction AI model and the disease monitoring AI model that have been previously used for disease prediction and disease monitoring. The AI model providing unit 820 may provide the disease prediction AI model and the disease monitoring Al model so that developers can utilize the disease prediction AI model and the disease monitoring AI model for updating and additionally training the AI model.

Although it is expressed as the AI model providing unit 820, various modules for disease prediction and disease analysis other than AI models may be provided by the AI model providing unit 820.

The developer workspace 830 may be implemented to develop a new AI model based on database-based training data and an existing AI model. The developer workspace 830 may be implemented to enable AI training to generate candidate AI models for newly replacing the AI models on the disease management device or to generate an AI model for predicting a new disease.

According to an embodiment of the present invention, in the developer workspace 830, when a keyword for a disease to be developed is input, training data related to input disease data may be recommended, and information on a target AI engine currently being used as an AI engine related to the input disease data may be provided.

The embodiments of the present invention described above may be implemented in the form of program instructions that can be executed through various computer units and recorded on computer readable media. The computer readable media may include program instructions, data files, data structures, or combinations thereof. The program instructions recorded on the computer readable media may be specially designed and prepared for the embodiments of the present invention or may be available instructions well known to those skilled in the field of computer software. Examples of the computer readable media include magnetic media such as a hard disk, a floppy disk, and a magnetic tape, optical media such as a compact disc read only memory (CD-ROM) and a digital video disc (DVD), magneto-optical media such as a floptical disk, and a hardware device, such as a ROM, a RAM, or a flash memory, that is specially made to store and execute the program instructions. Examples of the program instruction include machine code generated by a compiler and high-level language code that can be executed in a computer using an interpreter and the like. The hardware device may be configured as at least one software module in order to perform operations of embodiments of the present invention and vice versa.

While the present invention has been described with reference to specific details such as detailed components, specific embodiments and drawings, these are only examples to facilitate overall understanding of the present invention and the present invention is not limited thereto. It will be understood by those skilled in the art that various modifications and alterations may be made.

Therefore, the spirit and scope of the present invention are defined not by the detailed description of the present invention but by the appended claims, and encompass all modifications and equivalents that fall within the scope of the appended claims.

## Claims

1. A disease prediction and analysis method for disease management, the method comprising:
receiving, by a disease management device, user data; and
generating, by the disease management device, user disease management data based on the user data.

2. The method of claim 1, wherein the disease management data includes disease prediction data for a user or disease monitoring data for the user,
the disease prediction data is generated based on a disease prediction artificial intelligence (AI) model, and
the disease monitoring data is generated based on a disease monitoring Al model.

3. The method of claim 2, wherein the user data is preprocessed into user data (disease prediction) and input into the disease prediction AI model,
the user data is preprocessed into user data (disease monitoring) and input into the disease monitoring AI model, and
the user data (disease prediction) and the user data (disease monitoring) are time-series data.

4. A disease management device for performing disease prediction and disease analysis for disease management, the disease management device being implemented to:
receive user data, and
generate user disease management data based on the user data.

5. The disease management device of claim 4, wherein the disease management data includes disease prediction data for a user or disease monitoring data for the user,
the disease prediction data is generated based on a disease prediction AI model, and
the disease monitoring data is generated based on a disease monitoring Al model.

6. The disease management device of claim 5, wherein the user data is preprocessed into user data (disease prediction) and input into the disease prediction AI model,
the user data is preprocessed into user data (disease monitoring) and input into the disease monitoring AI model, and
the user data (disease prediction) and the user data (disease monitoring) are time-series data.
